(19)

Europäisches
Patentamt
European
Patent Office
Office européen
des brevets

(11) **EP 3 590 502 A1**

(12) # EUROPEAN PATENT APPLICATION

(43) Date of publication:
**08.01.2020 Bulletin 2020/02**

(21) Application number: **19183922.4**

(22) Date of filing: **02.07.2019**

(51) Int Cl.:
*A61K 9/14* (2006.01)     *A61K 9/16* (2006.01)
*A61K 9/19* (2006.01)     *A61K 31/137* (2006.01)
*A61P 11/10* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.07.2018   IT 201800006909**

(71) Applicant: **Hollycon Italy Pte. Ltd. - S.r.l.
06073 Corciano PG (IT)**

(72) Inventors:
• **Wu, Qi
   06073 Corciano (IT)**
• **Wu, Huaxin
   06073 Corciano (IT)**

(74) Representative: **Predazzi, Valentina et al
Società Italiana Brevetti S.p.A.
Piazza di Pietra, 39
00186 Roma (IT)**

(54) **AMBROXOL DRY POWDER FOR INHALATION USE WITH BRONCHIAL TARGET**

(57)     The present invention relates to a preparation for inhalation use in form of dry powder comprising particles of ambroxol and/or hydrochlorides thereof, and at least a pharmaceutically acceptable excipient and/or dispersant wherein at least 85% of said particles has a geometrical diameter from 3 to 5 μm. The present invention further relates to the methods for preparing such composition and uses thereof. The present invention thanks to the very fine particles of dry powder, as above defined, constitutes a composition for inhalation use with optimum performances which, combined with inhaler action, reaches and acts directly on the bronchi or on the damaged respiratory tract. The local action and the quick absorption of the drug guarantee good features of bioavailability. The decrease in dosing Ambroxol with respect to the other existing oral or injectable formulations allows to reach quickly, locally and safely, the therapeutic target.

EP 3 590 502 A1

## Description

The present invention relates to a preparation for inhalation use in form of dry powder comprising particles of Ambroxol and/or hydrochlorides thereof as single active principle and optionally a suitable pharmaceutically acceptable excipient, wherein said particles have a geometrical diameter equal to or smaller than 5 $\mu$m, wherein at least 85% of the particles of ambroxol and/or hydrochlorides thereof in the composition has a geometrical diameter from 3 to 5 $\mu$m. The present invention further relates to the methods for the preparation of such composition and uses thereof. The present invention thanks to the very fine particles of dry powder, having sizes equal to or smaller than 5 $\mu$m constitutes a composition for inhalation use with optimum performances which, combined to the inhaler action, reaches and acts directly on the bronchi or on the damaged respiratory tract. The local action and the quick absorption of the drug guarantee good features of bioavailability. The decrease in the dosage of Ambroxol with respect to the other existing oral or injectable formulations allows to reach locally and safely the therapeutic target.

STATE OR PRIOR ART

Ambroxol or most used hydrochlorides thereof, such as for example Ambroxol hydrochloride, is an expectorant mucolytic drug. Ambroxol is an active metabolite of bromhexine which can favour the secretion of pulmonary surfactants and the mucosal secretions of the respiratory tract. Ambroxol fragments the mucopolysaccharide chains in the sputum, it dissolves mucus, reduces significantly viscosity of the mucosal secretions, increases the mucociliary clearance and favours expectoration. It improves ventilation and breathing difficulties. Its effectiveness as mucolytic agent is significantly higher than that of bromhexine, with low toxicity and good tolerability. Drug variants are included in the European Pharmacopoeia (EP), in the United States Pharmacopoeia (USP), in the British Pharmacopoeia (BP), in the Chinese Pharmacopoeia (CP) and others.

Currently, Ambroxol still occupies a privileged role among the mucolytic agents, with several formulations and dosages available on the market, in form of tablets, capsules oral solutions, syrups, injections, liquid solutions for aerosol, etc. For adults and children over 12 years, the usual dose of oral administration: 30 mg-3 times a day; of intravenous injection, intramuscular injection and subcutaneous injection: 15 mg/twice a day; inhalation of liquid aerosol: 15 mg / 2 ml, twice a day. The oral preparations work slowly, they enter the gastrointestinal tract, they are absorbed by the blood flow to perform a systemic role and they cannot ease quickly the patient disease. The parental administration route, although it provides a quick therapeutic effect, plays a systemic role, it is relatively difficult to be administrated and causes pain in patient. The nebulized inhalants request the use of voluminous atomizers to atomize the liquid and then to inhale it, such devices are uncomfortable to be used and transported. The drug absorption rate by dry powder inhalation is second only to the intravenous injection. Especially for the diseases of bronchi and lungs it can act directly on the site of the lesions, which contributes to improve the bioavailability of the drugs and to exert a safe and effective therapeutic effect. At the same time, it favours stability and portability of the drugs.

Then, the need was very felt in the art for proposing new administration forms of Ambroxol and methods for the preparation thereof not having the disadvantages described in the known art.

SUMMARY OF THE INVENTION

Based upon the deficiencies of the several existing preparations and above-mentioned administration forms, the inventors succeeded to obtain a dry powder of Ambroxol and/or hydrochlorides thereof as single active principle and optionally a suitable pharmaceutically acceptable excipient, for inhalation use with bronchial target, with particles having a geometrical diameter equal to or smaller than 5 $\mu$m, wherein at least 85% of the particles of ambroxol and/or hydrochlorides thereof in the composition has a geometrical diameter from 3 to 5 $\mu$m. The present invention adopts a daily dosage form of dry powder for inhalation use equal to 1 up to 14.9 mg of the dry powder as defined in the present description and in the claims, in absence of propellants, with a good stability, with reduced interactions and interferences. The present invention will be preferably used with an inhaler device combined with blister or single-dose capsule, preferably blister made of aluminium, so that Ambroxol, through the inhaler, activated by the generation of an airflow due to the aspiration by the patient, could directly be inhaled to reach the bronchi and the damaged respiratory tracts, with a quick and safe effect.

The invention also relates to processes for the preparation of the dry powder comprising particles of ambroxol as defined in the present description and in the claims, and the products obtainable by means of said processes.

The present invention then relates to a method for preparing a pharmaceutical composition for inhalation use in form of dry powder of ambroxol and/or hydrochlorides thereof as single active principle, wherein at least 85% of the particles of ambroxol has a geometrical diameter from 3 to 5 $\mu$m comprising

a) to micronize ambroxol and/or hydrochlorides thereof by means of Jet Milling,

b) to collect the particles separated from the cyclone generated by said jet milling having a satisfying size, wherein at least 85% of the particles of ambroxol and/or hydrochlorides thereof has a geometrical diameter from 3 to 5 $\mu$m,

c) to mix the so obtained particles together with a suitable excipient thus obtaining the dry powder for inhalation use.

The present invention further relates to a method for preparing a pharmaceutical composition for inhalation use in form of dry powder of ambroxol and/or hydrochlorides thereof as single active principle, wherein at least 85% of the particles of ambroxol and/or hydrochlorides thereof has a geometrical diameter from 3 to 5 $\mu$m comprising

a. to heat and dissolve in distilled water or in distilled water containing ethanol $\leq$ 20% ambroxol and/or hydrochlorides thereof

b. to subject the preparation obtained in point a. to spray drying, by obtaining particles of ambroxol and/or hydrochlorides thereof wherein at least 85% of the particles of ambroxol and/or hydrochlorides thereof has a geometrical diameter from 3 to 5 $\mu$m,

c) to mix the so-obtained particles together with a suitable excipient thus obtaining the dry powder for inhalation use.

At last, the present invention relates to a method for preparing a pharmaceutical composition for inhalation use in form of dry powder of ambroxol and/or hydrochlorides thereof as single active principle, wherein at least 85% of the particles of ambroxol and/or hydrochlorides thereof has a geometrical diameter from 3 to 5 $\mu$m comprising:

a. to heat and dissolve in distilled water and/or hydrochlorides thereof and a suitable excipient

b. to subject the preparation obtained in point a. to spray freeze drying, obtaining particles of ambroxol and/or hydrochlorides thereof wherein at least 85% of the particles of ambroxol and/or hydrochlorides thereof has a geometrical diameter from 3 to 5 $\mu$m thus obtaining the dry powder for inhalation use.

As above said, any embodiment of the composition and of the medical devices provided in the present invention, can be implemented with the dry powder obtainable by anyone of the above-described or claimed methods.

The inhalation of dry powder is an effective method to act directly on bronchi and lungs. The dry powder of Ambroxol for inhalation use of the present invention, thanks to the reduced size of the particles and through the inhaler device reaches directly the site of bronchial lesion with a good *targeting* and a quick effect. The drug dosage is significantly reduced, it is safe and has reduced toxic and side effects.

The present invention constitutes a new administration route and a new dosage form of Ambroxol, it has the features of easy use and portability and it is an industrial product.

The hermetic insertion of the dry powder as single dose in blister made of aluminium or capsules guarantees an accurate dosage, excluding the overdosage thereof and with an optimum stability.

The inhalation of the drug in dry powder of the present invention, combined with an inhaler device also allows a patient with poor pulmonary function to use it, the procedure is simple, both elderly people and children can use it.

The advantages, features and use modes of the present invention will result evident from the following detailed description of some embodiments, shown by way of example and not for limitative purpose.

To the purpose of the present description, the term "geometrical diameter" has the meaning commonly used in the art, said geometrical diameter being determinable, for example, by means of the method shown by the European Pharmacopoeia (SPOS, Ph.Eur.9th Ed.,2.9.31 .Particle size analysis by laser light diffraction, precisely on page 349) through granulometer Accusizer ™ Optical Particle Sizer Model 770 (Santa Barbara, California, USA).

To the purpose of the present invention the expression "comprising" can be replaced by the term "constituted by" in any embodiment of the invention provided in the present description.

Therefore, in any embodiment of the invention, the pharmaceutical composition can be constituted by the described components.

The invention also relates to a therapeutic method for treating the pathologies illustrated in the present description, comprising the administration of the composition as defined in the present description, preferably by means of the device as defined in the present description to a subject requiring it.

DETAILED DESCRIPTION OF THE INVENTION

The present invention utilizes Ambroxol (trans-4-(2-Amino - 3,5-dibromobenzylamino) - cyclohexanol) and the hydrochlorides thereof as active principle, such as for example Ambroxol hydrochloride. The present invention firstly relates to a pharmaceutical composition for inhalation use in form of dry powder comprising particles of Ambroxol and/or hydrochlorides thereof as single active principle, and optionally a suitable pharmaceutically acceptable excipient, and wherein at least 85% of the particles of ambroxol and/or hydrochlorides thereof in said composition has a geometrical diameter from 3 to 5 $\mu$m.

The composition could include one or more excipients and/or dispersants and/or glidants suitable to the inhalation use, for example excipients could be used such as lactose, mannitol, glucose or mixtures thereof, one or more among solubilization agents, such as leucine and valine and/or glidants such as for example magnesium stearate. The percentages in the present description are meant as percentages by weight.

The composition could comprise from 20 to 100% of ambroxol, in particular from 20 to 59% or from 91 to 100% and/or from 41 to 80% of excipients and/or from 0 to 9% of dispersant. The dry powder of Ambroxol for inhalation use will be advantageously inserted hermetically as single dose in blister or capsules, preferably in blister made of aluminium. According to a preferred embodiment a self-piercing blister will be used for dispensing said powder, in particular like those described in the patent in Italy 102015000063968 herein incorporated by reference.

The present invention also relates to an inhaler of dry powder comprising the herein described compositions, advantageously an inhaler will be used activated by means of the air flow alone due to aspiration by the patient. In particular an inhalator will be used which can produce a turbulent flow speed so that, even under lower differential pressure conditions, it succeeds in disaggregating and dispersing particles of drug to form inhalable particles. Examples of suitable inhalers which could be used are PillHaler® or those described in the patent application in Italy 102016000093878 herein incorporated by reference.

The composition in dry powder of Ambroxol for inhalation use, the present invention relates to, will be used as expectorant mucolytic agent, in particular the herein described dry powder once inhaled is able to reach effectively the target of the bronchial lesions. The dry powder of Ambroxol according to the present invention could be used as single dose with dosage significantly lower than the several formulations on the market and which thanks to the very fine particles of dry powder having sizes included within $3 - 5 \mu m$ and the excipients for inhalation use and the dispersing agents, produces an inhalation dry product with optimum performances which, combined to the inhaler action, reaches and acts directly on the bronchi or on the damaged respiratory tract. The single dose of dry powder of Ambroxol will be preferably comprised from 1mg to 14.9 mg, which is only 1/2-1/6 of the several formulations on the market. The dosage regime could be for example one or twice a day.

The present invention also relates to the methods for preparing the herein described dry powder of Ambroxol and/or hydrochlorides thereof as well as the power obtainable by means of the said processes.

According to a first embodiment the method provides a step wherein the drug of Ambroxol and/or hydrochlorides thereof is micronized by pulverization of the air flow (jet milling), by obtaining the wished granulometry. According to an embodiment the used process gas will have a pressure of about 7 bar. The so prepared powder could be mixed uniformly with the excipients and/or the pharmaceutically acceptable dispersing agents.

The so obtained powder will be preferably inserted hermetically as single dose, in the amounts shown in the present description, in blister made of aluminium or capsules.

In a preferred embodiment of the invention mannitol is used as excipient.

According to an alternative embodiment the method provides a step a) of mixing Ambroxol and/or hydrochlorides thereof in distilled water or in distilled water and ethanol ≤20%.

The mixture prepared in step a) is subjected to a step b) of spray drying with the purpose of obtaining fine particles according to the wished granulometry, the particles obtained in step b) are then optionally mixed with one or more pharmaceutically acceptable excipients and/or dispersants.

The so obtained powder will be preferably inserted hermetically as single dose, in the amounts shown in the present description, in blister made of aluminium or capsules.

The prepared liquid is nebulized in small drops thanks to a nozzle or a rotating disc (in English nozzle or rotary atomizer). The nozzles use the pressure or the compression of a gas (for example air or nitrogen) to nebulize the prepared liquid whereas a disc atomizer uses a disc which rotates at high speed. The speed of the air and/or of the inert gases depends upon the section of the spray drier. This procedure is performed in the drying Chamber (the final size of the solid mainly depends upon the diameter of the drops produced by the atomizer). The produced drops are dried by hot air circulating in the drying chamber. The heated gas is put in contact with the small drops by using a gas spreader, thus by guiding the liquid evaporation (the contact between the nebulized drops and the hot air causes a quick evaporation of the solvent, rapidity which is determined by the very high contact area between the drops and the hot air).

During the spray drying step preferably an opening of the nozzle of the spray dryer of at least 0.7 mm and/or a gas flow between 200 and 800 L/h will be used. The temperature of inlet air preferably will be of at least 115± 2° C. The solution nebulization is made to occur for example with a liquid feeding speed of about ,8 ml/min. The so obtained powder could be mixed with excipient or inserted hermetically as single dose in blister made of aluminium or capsule in the amounts shown in the present description. The powder is recovered from the outletting gas for example by using a Cyclone or a filter (bag filter).

According to another embodiment the method provides a step a) of mixing Ambroxol and/or hydrochlorides thereof with dispersant and/or excipients in solution, for example in distilled heated water. The mixture prepared in step a) is subjected to a step b) of spray freeze drying with the purpose of obtaining fine particles according to the wished granulometry. According to a preferred embodiment an opening of the freeze dryer nozzle of at least 0,7 mm and/or a spray freezing temperature lower than or equal to -15° C and/or a freezing temperature (cryo-temperature) lower than or equal to -60° C and/or a liquid feeding speed of at least 1.6 ml/min and/or an atomizing pressure of at least 2.7 bar will be used.

The heating temperatures preferably are 70-75°C when the distilled water is heated and 50-55°C when the hydroalcoholic solution containing distilled water and ethanol ≤20% is heated.

The so obtained powder could be inserted hermetically as single dose, in the amounts shown in the present description, in blister made of aluminium or capsule.

The invention is described hereinafter in details in the following examples which are only by way of example without limiting the conferred protective scope.

EXAMPLES

Example 1

In this preparation method the micronizer is used: DECMCOne model. The drug of Ambroxol hydrochloride is micronized by pulverization of the airflow.

The air flow pulverizer uses a "process gas" 7 bar 0.09 Nm3/ min (3.18 CFM) the ambient temperature in white chamber is 20°C and the temperature in the area dedicated to the micronizer unit is ≤12°C. Through grinding and crushing at high pressure and the centrifugal force generated by the turbine, the rough particles are separated from the fine ones. The particles satisfying the size requirements enter the cyclone separator and are herein collected. It is preferred that at least 85% of particles has a diameter smaller than 5 μm, preferably from 3 to 5 μm. Reproducing and distributing uniformly excipient and dispersant in an inhalation level from 50 to 100 μm to obtain a good sliding capability. During inhalation the flow generates turbulences which disperse the particles to form micronized particles which can be inhaled to reach the bronchi. The percentage by weight ratio of the particles of active principle and the particles of excipient is 40%:60%. The deriving powder is inserted hermetically as single dose in blister made of aluminium or capsules.

The excipient used in the above-mentioned example preferably is mannitol.

Example 2

In this preparation method the mini spray dryer BUCHI B-290 is used. The opening of the spray dryer nozzle: 0.7 mm, compressed gas flow: 5 ∼ 8bar, 200 ∼ 800 L / h, temperature of inlet air 115± 2° C, liquid feeding speed: 1.8 ml / min, nebulization air speed: 473 litres/hour, atomization pressure: 2 bar, air flow: 0.55 m3/min, drying time ≤ 1.5-seconds. Ambroxol and/or hydrochlorides thereof are dissolved in distilled water comprising or not 20% of ethanol, the spray-drying method is used with the solution obtained by adjusting the nebulization air speed to control the size of the particles of atomized small drops to prepare particles of micronized powder with a good form. It is preferred that at least 85% of particles has a diameter smaller than 5 μm, preferably from 3 to 5 μm and that 40% of the particles of excipient has a diameter from 15 to 100 μm mixed uniformly to obtain a good sliding capability. During inhalation the flow generates turbulences which disperse the particles to form micronized particles which can be inhaled to reach the bronchi. The deriving powder is inserted hermetically as single dose in blister made of aluminium or capsules.

Example 3

In this preparation method the freeze dryer Pilotech YC-3000 is used.

Opening of the freeze dryer nozzle: 0.7 mm, temperature of spray freezing: ≤ -15° C, cryo-temperature: ≤ -60° C, liquid feeding speed 1.6 ml / min, atomization pressure: 2.7 bar. Based upon the percentage by weight of the components of the end mixture, 91% of Ambroxol hydrochloride and 9% of mannitol are dissolved in distilled heated water. The size of the particles of the atomized small drops is controlled by freeze dryer method to prepare particles of micronized powder with a good form. It is preferred that at least 85% of particles has a geometrical diameter from 3 to 5 μm. The particles are mixed uniformly with ≤0.5% of dispersant to obtain a good sliding capability. During inhalation the flow generates turbulences which disperse the particles to form micronized particles which can be inhaled to reach the bronchi. The deriving powder is inserted hermetically as single dose in blister made of aluminium or capsules.

Example 4

Determination of the physical and chemical properties of the powder
The physical and chemical properties of the dry powder for inhalation use obtained with the herein described preparation methods, the present invention relates to, were determined by meeting the standards related to the European Pharmacopoeia (EP). Determination of the water content by thermogravimetric analysis (Ph. Eur. 9th Ed, 2.2 Thermal analysis). Morphological characterization of the powder: characterization of the surface morphology of the powder by means of scanning electron microscopy. Several preparation methods generate different surface morphologies of the powder. The product prepared with the micronization method has an irregularly wrinkled aspect, whereas the one obtained through spray drying process will have a spherical shape. Size characterization of the particles: the particle sizes were determined with the method illustrated by the European Pharmacopoeia (SPOS, Ph.Eur.9th Ed.,2.9.31. Particle size analysis by laser light diffraction, described in detail on page 349) through granulometer Accusizer ™ Optical Particle Sizer Medel 770 (Santa Barbara, California, USA). 85% of the obtained powder particles has a diameter smaller than 5 μm, preferably from 3 to 5 μm. Meas-

urement of the aerodynamic diameter of the powders by cascade impactor (Ph. Eur. 9th Ed., 2.9.18. Preparations for inhalation: aerodynamic assessment of fine particles). Study of the sliding properties and density of the powders by measuring the tap density and angle of repose according to the European Pharmacopoeia (Ph. Eur. 9th Ed., 2.9.34. Bulk density and tapped density of powders; 2.9.36. Powder flow].

Example 5

Development and validation of a UV (Ph. Eur. 9th Ed., 2.2.25. Spectrophotometry, ultraviolet and visible absorption) or HPLC (Ph. Eur. 9th Ed.,2.2.29. Liquid chromatography) method for the quantitative determination of Ambroxol hydrochloride. The content of Ambroxol hydrochloride is 95%-105% with respect to the illustrated amount.

Example 6

Determination of the content uniformity of the prepared mixtures (Ph. Eur. 9th Ed. 2.9.40. Uniformity of dosage units).

Example 7

Determination of the properties of the dry powder for inhalation use.

Determination of the emptying speed:

Determination of the powder fraction emitted by using the apparatus *Glass Twin Stage Impinger* (Ph. Eur. 9th Ed., 2.9.18. *Preparations for inhalation: aerodynamic assessment of fine particles*) by following the guidelines of EMA. A dry powder inhaler device will be for example used which activates only thanks to the air flow alone generated by the patient's inhalation, including inside blister made of aluminium the formulations obtained with the herein described preparation methods.

10 accurately weighed products (W1) are collected. The inhalation of one product at a time is measured at an air flow di 60L ± 5L for 5 seconds to identify then each weight thereof (W2). Then, any residue is eliminated to obtain the value of the tare (W3). Through the following formula the value of the fraction of emitted powder will be obtained:

$$[(W1-W2) / (W1-W3)] \times 100\%.$$

Fraction of powder emitted by the product of the present invention: from 91 to 100%.

Determination of the pulmonary deposit through FPF analysis:

Glass Twin Stage Impinger (Ph. Eur. 9th Ed., 2.9.18. Preparations for inhalation: aerodynamic assessment of fine particles) of distribution of the dry powder for inhalation use, to measure the deposition level of Ambroxol hydrochloride in the simulated respiratory and pulmonary tract. By using for example a dry powder inhaler device which activates thanks to the air flow alone generated by the patient's inhalation, containing inside blister made of aluminium the formulations obtained with the herein described preparation methods. The content of Ambroxol hydrochloride is determined and the device is validated through high performance liquid chromatography (HPLC).

10 samples of this product are collected and the dry powder inhaler device is used, by using for example a dry powder inhaler device which activates thanks to the air flow alone generated by the patient's inhalation, containing inside blister made of aluminium the formulations obtained with the herein described preparation methods, the results are compared to the shown amount of this product to obtain the powder distribution quantity. The FPF deposit rate of this product is 0-50%.

Conclusion: From the evaluation of the dynamic particles of the product it is clear that the powder particles of the present invention are suitable for the administration to the bronchi. The selected excipients and their ratio with the drug show a good disaggregation level of the particles and then an optimum drug release. Most particles succeed in reaching the bronchial site.

Example 8

Study of the effectiveness of the Ambroxol hydrochloride on the bronchi.

To 18 laboratory rats, weighing 250g, divided into two groups, three doses of product were administered separately and diluted in the respective dose for injection of Ambroxol hydrochloride. The plasmatic concentration and the effect on the bronchi of the same dose of product for injection of Ambroxol hydrochloride is thus determined. Test methodology: all rats were anaesthetized with isoflurane, one group received separately three doses of product by using a micronized atomizer of animal lung similar to a syringe. A method for the direct administration of a nebulized powder to a trachea of a rat with the needle and the other group was subjected to intravenous injection of the same dose of Ambroxol hydrochloride by injection. Then, from the eye socket of the animal blood was collected to determine the plasmatic concentration in 15 minutes of the same dose of product and of the same dose by injection in the same time. From the drug concentration in the blood the absorption speed and level of the inhaled product can be evaluated, with the purpose of deducing the product effect and target. The experimental results show that the product target has been reached, the targeted administration of the drug is effective and allows to reduce the dose thereof, thus reducing the side effects. The container for the drug, used for implementing the present invention, preferably is in blister made of hermetic aluminium, as the dry powder preparation can be pre-packaged in an established single dose and inserted in the blister made of sealed aluminium by guaranteeing the powder tightness and its chemical-physical protection. Such packaged formulations can remain stable for a long period of time and they are also very advantageous from the economic and commercial point of view.

The inhalation device used for the administration of the present invention is a tool for inhaling dry powder PillHaler® which is fed exclusively by the inspiratory air flow of the patient, in combination with the blister made of sealed aluminium to form a unique product. The drug powder is effectively dispersed in the turbulence of the air flow produced by the inhalation of the patient and it is easily inhaled in the body. Even the patients with poor lung functionality can use it as the procedure is simple. It can be easily used by elderly people and children.

The present invention also relates to all products obtainable according to anyone of the herein described embodiments of the methods.

**Claims**

1. A pharmaceutical composition for inhalation use in form of dry powder comprising particles of Ambroxol and/or hydrochlorides thereof as single active principle, and at least a pharmaceutically acceptable excipient and/or dispersant wherein at least 85% of said particles of Ambroxol in the composition has a geometrical diameter between 3 and 5 μm.

2. The composition according to claim 1 wherein the amount of Ambroxol and/or hydrochlorides thereof per dosage unit is from 1 to 14.9 mg.

3. The composition according to anyone of claims 1 or 2 comprising one or more excipients and/or dispersants and/or glidants.

4. The composition according to anyone of claims 1 to 3 comprising lactose, mannitol, glucose, leucine, valine, magnesium stearate and/or mixtures thereof.

5. The composition according to anyone of claims 1 to 6 comprising 20 to 100%, in particular 20 to 59% or 91 to 100% and 91 to 100% of Ambroxol and/or 41 to 80% of excipients and/or 0 to 9% of dispersant and/or glidant.

6. The composition according to anyone of claims 1 to 5 for use as mucolytic expectorant agent, in particular for use as mucolytic expectorant agent acting directly on the site of the bronchial lesions.

7. A blister or capsule made of aluminium comprising a composition according to anyone of claims 1 to 6 inserted hermetically as single dose, in particular in a self-piercing blister.

8. A dry dust inhaler device comprising a composition according to anyone of claims 1 to 6 or a blister or capsule according to claim 7.

9. The inhaler device according to claim 8 **characterized in that** it is activated by the air flow alone due to the aspiration by the patient and/or **in that** it is able to produce a turbulent flow speed so that, even under lower differential pressure conditions, it succeeds in disaggregating and dispersing drug particles to form inhalable particles.

10. A method for preparing a composition comprising particles of Ambroxol and/or hydrochlorides thereof as single active principle, and at least a pharmaceutically acceptable excipient and/or dispersant wherein at least 85% of said particles of Ambroxol in the composition has a geometrical diameter from 3 to 5 μm, in form of dry powder comprising a first step a) of micronizing ambroxol and/or hydrochlorides thereof by air jet grinding and a second step b) wherein the dry powder obtained in step a) is mixed with one or more pharmaceutically acceptable excipients and/or dispersing agents.

11. A method for preparing a composition comprising particles of Ambroxol and/or hydrochlorides thereof as single active principle, and at least a pharmaceutically acceptable excipient and/or dispersant wherein at least 85% of said particles of Ambroxol in the composition has a geometrical diameter from 3 to 5 μm, in form of dry powder comprising a step wherein an aqueous solution or a hydroalcoholic solution of ethanol ≤20% of ambroxol and/or hydrochlorides thereof is subjected to a spray drying and a second step b) wherein the dry powder obtained in step a) is mixed with one or more pharmaceutically acceptable excipients and/or dispersing agents.

12. A method for preparing a composition comprising particles of Ambroxol and/or hydrochlorides thereof as single active principle, and at least a pharmaceutically acceptable excipient and/or dispersant wherein at least 85% of said particles of Ambroxol in the composition has a geometrical diameter from 3 to 5 μm, in form of dry powder comprising a step wherein an aqueous solution of ambroxol and/or hydrochlorides thereof and at least a pharmaceutically acceptable excipient is subjected to spray freeze drying.

13. The method according to anyone of claims 10 to 12 further comprising an additional step wherein said dry powder is inserted hermetically in an optionally self-piercing blister or capsule made of aluminium as single dose.

14. A pharmaceutical composition in form of dry powder for inhalation use in form of dry powder obtainable by means of the method according to anyone of claims 11 to 13.

15. The pharmaceutical composition for use according to claim 14 as defined in anyone of claims 2 to 7.

16. A blister or capsule made of aluminium comprising a composition according to anyone of claims 14 or 15 inserted hermetically as single dose, in particular in a self-piercing blister.

17. A dry powder inhaler device comprising a composition according to anyone of claims 14 or 15 or a blister or capsule according to claim 16.

Europäisches
Patentamt

European
Patent Office

Office européen
des brevets

# EUROPEAN SEARCH REPORT

Application Number

EP 19 18 3922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YACHAO REN ET AL: "Influence of Formulation and Preparation Process on Ambroxol Hydrochloride Dry Powder Inhalation Characteristics and Aerosolization Properties", DRUG DEVELOPMENT AND INDUSTRIAL PHARMACY, vol. 34, no. 9, 20 October 2008 (2008-10-20), pages 984-991, XP055568182, US ISSN: 0363-9045, DOI: 10.1080/03639040802154913 * abstract * * page 984, left-hand column, paragraph 1 - page 986, left-hand column, paragraph 5 * * page 987, right-hand column, paragraph 4 * * page 988; table 3 * ----- | 1-11 | INV. A61K9/14 A61K9/16 A61K9/19 A61K31/137 A61P11/10 |
| | -/-- | | |

TECHNICAL FIELDS SEARCHED (IPC)

A61K
A61P

The present search report has been drawn up for all claims

| Place of search | Date of completion of the search | Examiner |
|---|---|---|
| The Hague | 30 October 2019 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 19 18 3922

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SIE HUEY LEE ET AL: "A novel inhaled multi-pronged attack against respiratory bacteria", EUROPEAN JOURNAL OF PHARMACEUTICAL SCIENCES, vol. 70, 19 January 2015 (2015-01-19), pages 37-44, XP055568187, NL ISSN: 0928-0987, DOI: 10.1016/j.ejps.2015.01.005 * abstract * * page 38, right-hand column, last paragraph - page 39, right-hand column, paragraph 1 * * page 39, right-hand column, last paragraph * * page 40; figure 1 * * page 41; table 1 * * page 42; table 2 * | 1,3-9 | |
| X | CN 101 214 227 A (UNIV SHENYANG PHARMACEUTICAL [CN]) 9 July 2008 (2008-07-09) * the whole document * | 1-9 | TECHNICAL FIELDS SEARCHED (IPC) |
| X | CN 102 614 123 A (INST PHARM & TOXICOLOGY AMMS) 1 August 2012 (2012-08-01) * the whole document * | 1-9, 12-17 | |
| X | WO 2013/128283 A2 (ICEUTICA HOLDINGS INC BVI [GB]) 6 September 2013 (2013-09-06) * page 4, paragraph 1 * * page 81, last paragraph - page 85, paragraph 1 * * claims 1-79 * | 1,3-5, 7-9 | |

-/--

The present search report has been drawn up for all claims

| Place of search | Date of completion of the search | Examiner |
|---|---|---|
| The Hague | 30 October 2019 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 19 18 3922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MEER SAIFUL HASSAN ET AL: "Effect of Particle Formulation on Dry Powder Inhalation Efficiency", CURRENT PHARMACEUTICAL DESIGN, vol. 16, no. 21, 1 July 2010 (2010-07-01), pages 2377-2387, XP055568139, NL ISSN: 1381-6128, DOI: 10.2174/138161210791920423 * the whole document * | 1-17 | |
| X,P | WO 2018/148113 A1 (STC UNM [US]) 16 August 2018 (2018-08-16) * page 17, paragraph 2 - page 18, paragraph 2 * * page 21, paragraphs 3,4 * * claims 1-61 * | 1,3-5, 7-9, 12-17 | |

TECHNICAL FIELDS
SEARCHED (IPC)

The present search report has been drawn up for all claims

| Place of search | Date of completion of the search | Examiner |
|---|---|---|
| The Hague | 30 October 2019 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 18 3922

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2019

| Patent document cited in search report | | Publication date | Patent family member(s) | | Publication date |
|---|---|---|---|---|---|
| CN 101214227 | A | 09-07-2008 | NONE | | |
| CN 102614123 | A | 01-08-2012 | NONE | | |
| WO 2013128283 | A2 | 06-09-2013 | AU | 2013227351 A1 | 18-09-2014 |
| | | | AU | 2016202881 A1 | 26-05-2016 |
| | | | AU | 2017213466 A1 | 31-08-2017 |
| | | | CA | 2865702 A1 | 06-09-2013 |
| | | | CN | 104703584 A | 10-06-2015 |
| | | | CN | 107875127 A | 06-04-2018 |
| | | | EP | 2819645 A2 | 07-01-2015 |
| | | | HK | 1210706 A1 | 06-05-2016 |
| | | | IL | 234365 A | 26-09-2019 |
| | | | JP | 6231022 B2 | 15-11-2017 |
| | | | JP | 2015508808 A | 23-03-2015 |
| | | | KR | 20150041608 A | 16-04-2015 |
| | | | NZ | 629438 A | 28-10-2016 |
| | | | NZ | 722952 A | 21-12-2018 |
| | | | RU | 2014139046 A | 20-04-2016 |
| | | | RU | 2019110128 A | 08-05-2019 |
| | | | SG | 10201703243Q A | 29-06-2017 |
| | | | SG | 11201405243T A | 26-09-2014 |
| | | | US | 2014065219 A1 | 06-03-2014 |
| | | | US | 2018318170 A1 | 08-11-2018 |
| | | | WO | 2013128283 A2 | 06-09-2013 |
| WO 2018148113 | A1 | 16-08-2018 | AU | 2018219149 A1 | 19-09-2019 |
| | | | CA | 3055311 A1 | 16-08-2018 |
| | | | KR | 20190116320 A | 14-10-2019 |
| | | | US | 2019269806 A1 | 05-09-2019 |
| | | | WO | 2018148113 A1 | 16-08-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102015000063968

- IT 102016000093878

**Non-patent literature cited in the description**

- Preparations for inhalation: aerodynamic assessment of fine particles. Ph. Eur.